# EUROPÄISCHE PATENTSCHRIFT

(11) **EP 1 551 320 B1**
(45) Veröffentlichungstag und Bekanntmachung des Hinweises auf die Patenterteilung: **07.12.2005**
(21) Anmeldenummer: 02807751.9
(22) Anmeldetag: 04.09.2002
(51) Int. Cl.: A61B 17/70

(54) **ORTHOPÄDISCHE FIXATIONSEINRICHTUNG**
ORTHOPEDIC FIXATION DEVICE
DISPOSITIF DE FIXATION ORTHOPEDIQUE

(43) Veröffentlichungstag der Anmeldung: 13.07.2005
(73) Patentinhaber: Aesculap AG & Co. KG, 78532 Tuttlingen (DE)
(72) Erfinder: KRAMER, Ulrich, 78532 Tuttlingen (DE); SCHUMACHER, Jörg, 78532 Tuttlingen (DE); KOZAK, JEFFREY, HOUSTON, TX 77030-4509 (US); BEISSE, RUDOLF, 82418 SEEHAUSEN (DE); POTULSKI, MICHAEL, 82441 OHLSTADT (DE)
(74) Vertreter: Boehme, Ulrich
(86) Internationale Anmeldenummer: PCT/EP2002/009878
(87) Internationale Veröffentlichungsnummer: WO 2004/021901

(56) Entgegenhaltungen:
- EP-A- 0 384 001
- US-A- 5 330 473
- US-A- 5 611 800

## Beschreibung

Die Erfindung betrifft eine orthopädische Fixationseinrichtung zur Festlegung eines stabförmigen Fixierelementes mit zwei gegeneinander bewegbaren, bei Annäherung das Fixierelement zwischen sich einspannenden Klemmbacken.

Zur Fixierung von Knochen oder Knochenteilen relativ zueinander, beispielsweise im Bereich der Wirbelsäule, ist es bekannt, stabförmige Fixierelemente zu verwenden, die an verschiedenen Stellen zwischen Klemmbacken eingespannt sind, diese Klemmbacken werden dann über Knochenschrauben oder ähnliche Fixiermittel mit den zu fixierenden Knochen oder Knochenfragmenten verbunden (WO 97/06742; US 5,741,255; US 5,676,703; US 5,024,213; US 5,474,551). Dabei treten zum Teil sehr hohe Kräfte auf, so daß diese Fixationseinrichtungen sehr stabil gebaut werden müssen. Dies führt zwangsläufig dazu, daß bekannte Vorrichtungen zum Teil kompliziert und sperrig aufgebaut sind. Insbesondere bei minimalinvasivem Zugang ist es daher schwierig, diese Fixationseinrichtungen zu implantieren und die verschiedenen Klemmschrauben und andersartigen Klemmechanismen zu betätigen. Teilweise müssen mehrere Schrauben oder Klemmuttern angezogen werden (US 5,741,255), teilweise müssen die Einzelteile im Körper zusammengefügt werden, da Schraubrichtung und Einschubrichtung der stabförmigen Fixierelemente unterschiedlich sind (WO 97/06742; US 5,474,551).

Es ist Aufgabe der Erfindung, eine gattungsgemäße Fixationseinrichtung so auszubilden, daß sie in einfacher Weise implantiert und fixiert werden kann.

Diese Aufgabe wird bei einer orthopädischen Fixationseinrichtung der eingangs beschriebenen Art erfindungsgemäß dadurch gelöst, daß in Spannrichtung neben den Klemmbacken ein Exzenterkörper drehbar an der Fixationseinrichtung gelagert ist, der beim Verdrehen einen Klemmbacken gegen den anderen verschiebt.

Bei einer solchen Einrichtung ist es ohne weiteres möglich, das stabförmige Fixierelement von oben her zwischen die beiden Klemmbacken einzuschieben und diese dann einfach dadurch zu fixieren, daß der Exzenterkörper verdreht wird. Komplizierte Schraubvorgänge mit vielen Umdrehungen sind auf diese Weise nicht notwendig, so daß auch durch kleine Körperöffnungen diese Vorgänge ohne weiteres erfolgen können. Die Handhabung des Exzenterkörpers kann zusätzlich dadurch vereinfacht werden, daß die Verdrehmöglichkeit des Exzenterkörpers begrenzt ist, beispielsweise durch Anschläge in der Spannrichtung und/oder in der Löserichtung. Dadurch wird einerseits dem Operateur signalisiert, ob eine vollständige Spannung oder Lösung des Exzenterkörpers stattgefunden hat, zum anderen wird eine Überspannung verhindert, die gegebenenfalls zu Beschädigungen der Einrichtung führen könnte. Die Begrenzung kann durch Anschläge vorgenommen werden.

Bei einer bevorzugten Ausführungsform ist vorgesehen, daß der Exzenterkörper auf einem rotationssymmetrischen Lagerkörper drehbar gelagert ist, an dem auch die Klemmbacken gehalten sind. Die Drehachse des Exzenterkörpers kann vorzugsweise parallel zur Achse des rotationssymmetrischen Lagerkörpers verlaufen, insbesondere ist die Drehachse des Exzenterkörpers mit der Achse des rotationssymmetrischen Lagerkörpers identisch.

Der rotationssymmetrische Lagerkörper kann beispielsweise kreiszylindrisch ausgebildet sein, es ist aber auch möglich, diesen Lagerkörper z.B. kugelig oder konisch auszubilden. Besonders vorteilhaft ist es, wenn der Lagerkörper durch den Schaft einer Knochenschraube oder eines Hakens_gebildet wird, es genügt dann nämlich, auf diesen Schaft Klemmbacken und Exzenterkörper aufzuschieben und den Exzenterkörper um den Lagerkörper zu verdrehen, um die Fixationseinrichtung zusammenzubauen und zu fixieren. Diese Fixierung kann dabei sowohl eine Fixierung gegenüber einer Verdrehung als auch gegenüber einer axialen Verschiebung sein.

Es ist vorteilhaft, wenn an dem rotationssymmetrischen Lagerkörper ein Träger gehalten ist, der die beiden Klemmbacken trägt.

Bei einer ersten bevorzugten Ausführungsform ist der Träger starr mit dem Lagerkörper verbunden, bei einer solchen Ausgestaltung sind die Klemmbakken im wesentlichen nur in Klemmrichtung gegeneinander verschiebbar.

Bei einer bevorzugten Ausführungsform ist der Träger jedoch um die Symmetrieachse des rotationssymmetrischen Lagerkörpers verdrehbar an diesem gehalten. Man erhält auf diese Weise eine weitere Einstellungsmöglichkeit, die Klemmbacken können also nicht nur gegeneinander gespannt werden, sondern gemeinsam auch um die Achse des Lagerkörpers verdreht werden. Dabei ergibt sich bei dieser Ausgestaltung zwangsläufig beim Verdrehen des Exzenterkörpers auf dem Lagerkörper nicht nur ein Festklemmen des stabförmigen Fixierelementes zwischen den Klemmbacken, sondern zusätzlich auch eine Festlegung des verdrehbar auf dem Lagerkörper gelagerten Trägers, da der Exzenterkörper sich an dem Lagerkörper abstützt und den Träger dadurch gegenüber dem Lagerkörper verklemmt.

Der Träger kann in Richtung der Drehachse auf den rotationssymmetrischen Lagerkörper aufschiebbar sein, so daß sich eine besonders einfache Montage des Implantats ergibt, es genügt nämlich beispielsweise bei einem Lagerkörper, der durch den Schaft einer Knochenschraube gebildet wird, den Träger von oben her auf die Knochenschraube aufzuschieben, in Aufschubrichtung kann dann auch ein Werkzeug eingeführt werden, mit dem der Exzenterkörper verstellt wird, dazu ist nur eine sehr kleine Körperöffnung notwendig.

Bei einer bevorzugten Ausführungsform ist der eine der beiden Klemmbacken als fester Klemmbacken ausgebildet, dessen Abstand von der Drehachse des Exzenterkörpers im wesentlichen fest ist, während der andere als beweglicher Klemmbacken ausgebildet ist, der durch den Exzenterkörper gegen den festen Klemmbacken bewegbar ist.

Grundsätzlich kann der feste Klemmbacken starr mit dem Träger verbunden sein. Besonders vorteilhaft ist jedoch eine Ausgestaltung, bei welcher die Klemmbacken um eine parallel zur Klemmrichtung der Klemmbacken verlaufende Drehachse verschwenkbar mit dem Träger verbunden sind. Hier ergibt sich eine weitere Verstellmöglichkeit, so daß auch die Richtung der stabförmigen Fixierelemente relativ zu dem Lagerkörper verstellbar ist. Es ist dabei vorteilhaft, wenn am Träger ein Anschlag für den festen Klemmbacken angeordnet ist, der eine Verschiebung des festen Klemmbackens in Richtung der Drehachse des Klemmbackens und damit eine Entfernung des festen Klemmbackens von der Drehachse des Exzenterkörpers begrenzt. Bei einer solchen Ausgestaltung sind die Klemmbacken gegenüber dem Träger um die parallel zur Klemmrichtung verlaufende Drehachse frei verdrehbar, solange der Exzenterkörper nicht gespannt ist. Spannt man jedoch den Exzenterkörper, werden die Klemmbacken gemeinsam gegen den Anschlag geschoben und damit gegen eine weitere Verdrehung um die parallel zur Klemmrichtung verlaufende Drehachse fixiert, der bewegliche Klemmbacken wird dann weiter gegen den nunmehr durch den Anschlag festgehaltenen Klemmbacken verschwenkt und klemmt zusätzlich das stabförmige Fixierelement zwischen den Klemmbacken ein. Man kann also allein durch Spannen des Exzenterkörpers alle Freiheitsgrade beseitigen, die die Anordnung bei gelöstem Exzenterkörper aufweist.

Die Drehachse der Klemmbacken und die Drehachse des Exzenterkörpers können in einer Ebene liegen, bei einer abgewandelten Ausführungsform kann aber auch vorgesehen sein, daß die Drehachse der Klemmbacken und die Drehachse des Exzenterkörpers seitlich gegeneinander versetzt sind. Durch diese unterschiedlichen Anordnungen läßt sich das Implantat an die örtlichen Gegebenheiten anpassen.

Es ist günstig, wenn beide Klemmbacken durch die Seitenwände eines im Querschnitt U-förmigen Lagerkörpers gebildet werden, dessen Seitenwände federnd gegeneinander biegbar sind. Dabei ergibt sich eine besonders günstige Ausgestaltung, wenn die Klemmbacken bei gelöstem Exzenterkörper federnd soweit auseinander bewegbar sind, daß ein stabförmiges Fixierelement zwischen sie einschiebbar ist. Dadurch ist ein elastisches Einschnappen des Fixierelementes zwischen die beiden Klemmbacken möglich, solange der Exzenterkörper noch nicht gespannt ist, trotzdem werden die federnden Klemmbacken das eingelegte Fixierelement vorläufig fixieren, so daß sich die Verbindung nicht sofort wieder löst, daß aber andererseits eine Justierung der Verbindung noch möglich ist.

Günstig ist es, wenn die Drehverbindung zwischen den Klemmbacken einerseits und dem Träger andererseits lösbar ist.

Beispielsweise kann die Drehverbindung zwei bajonettartig einander hintergreifende Lagerglieder umfassen, die sich in einer bestimmten Winkelstellung nicht hintergreifen und dann axial gegeneinander verschiebbar sind. Verdreht man beispielsweise Träger und Klemmbacken um 90° gegeneinander, könnte sie axial gegeneinander verschiebbar sein, in einer parallelen Ausrichtung dagegen sind beide Lagerglieder in axialer Richtung aneinander festgelegt, so daß dann eine Drehverbindung entsteht, die über einen bestimmten Winkelbereich nicht gelöst werden kann, sondern nur dann, wenn die beiden Lagerglieder wieder in die senkrecht zueinander verlaufende Ausgangsstellung verschwenkt werden.

Der Exzenterkörper kann unmittelbar an dem beweglichen Klemmbacken anliegen, es ist aber unter Umständen vorteilhaft, wenn zwischen dem Exzenterkörper und den Klemmbacken ein Zwischenglied angeordnet ist, welches beim Verdrehen des Exzenterkörpers mehr oder weniger gegen die Klemmbacken verschoben wird. Damit kann einerseits eine Anpassung der Dimensionierung erreicht werden, andererseits kann dieses Zwischenglied so ausgebildet sein, daß bei gegenüber dem Träger verdrehbaren Klemmbacken in unterschiedlicher Winkelstellung der Klemmbacken sichergestellt ist, daß der Exzenterkörper über das Zwischenglied die Spannkraft auf den beweglichen Klemmbacken übertragen kann.

Das Zwischenglied kann beweglich an dem Träger gehalten sein, insbesondere ist es günstig, wenn das Zwischenglied über eine federnde Verbindung an dem Träger gehalten ist.

Der Exzenterkörper kann als Ring ausgebildet sein.

Es ist günstig, wenn der Exzenterkörper Ausnehmungen zur Aufnahme eines Drehwerkzeuges aufweist, beispielsweise randseitige Rücksprünge, in die Vorsprünge eines Drehwerkzeuges formschlüssig eingreifen können.

Wie bereits erwähnt kann der Lagerkörper Teil einer Knochenschraube oder eines Hakens sein, beispielsweise kann der Lagerkörper durch einen zylindrischen Schaft einer Knochenschraube oder eines Hakens gebildet werden.

Eine besonders günstige Ausgestaltung ergibt sich dann, wenn der Lagerkörper über einen Querträger mit einer Knochenschraube verbunden ist, deren Längsachse parallel zur Längsachse des Lagerkörpers und seitlich versetzt zu dieser verläuft. Durch einen solchen Querträger ergibt sich gegenüber einer in den Knochen eingeschraubten Knochenschraube eine zusätzliche Variationsmöglichkeit der Positionierung des Lagerkörpers, der Querträger kann nämlich auf der Knochenschraube verdreht werden und in unterschiedliche Winkelstellung weisen oder auch in axialer Richtung verschoben werden, so daß eine optimale Anpassung an die anatomischen Gegebenheiten möglich wird. Insbesondere ist dabei vorteilhaft, wenn die Knochenschraube im Querträger um ihre Längsachse verdrehbar und in einer bestimmten Winkelstellung und/oder in einer bestimmten Axialposition festlegbar ist. Diese Festlegung kann vorzugsweise durch Klemmung erfolgen.

Zur Festklemmung im Querträger kann gemäß einer bevorzugten Ausführungsform der Erfindung ein Klemmstück gegen die Knochenschraube verschiebbar sein.

Einen besonders einfachen Aufbau erhält man, wenn zur Verschiebung des Klemmstückes im Querträger ein am Klemmstück anliegender, um eine Drehachse verdrehbarer Exzenter gelagert ist. Verdreht man diesen Exzenter, so verschiebt er das Klemmstück in die Klemmstellung, und die Knochenschraube und der Querträger sind nicht mehr gegeneinander verdrehbar, sondern fest miteinander verbunden. Auch hier kann die Drehbewegung des Exzenters begrenzt sein, insbesondere durch Anschläge, so daß eine Überspannung vermieden und außerdem die Handhabung für den Operateur erleichtert wird.

Die Drehachse des Exzenters steht vorzugsweise senkrecht auf der Ebene des Querträgers.

Die Drehachsen des Exzenters im Querträger und des Exzenterkörpers können parallel zueinander verlaufen, insbesondere können diese Drehachsen zusammenfallen.

Dabei können die Drehachse des Exzenters und die Längsachse der Knochenschraube parallel angeordnet sein, es ist aber bei einer besonderen Ausführungsform auch möglich, daß die Knochenschraube durch eine kugelige Verbindung mit dem Querträger verschwenkbar verbunden ist und das Klemmstück beim Fixieren in Richtung auf eine kugelige Lagerfläche der Knochenschraube verschiebbar ist. In diesem Falle kann also Knochenschraube gegenüber der Drehachse des Exzenters verschwenkt werden.

Eine besonders einfache Ausgestaltung ergibt sich, wenn der Querträger ein Langloch aufweist, in welchem die Knochenschraube, das Klemmstück und der Exzenter aufgenommen sind. Durch Verdrehen des Exzenters werden dieser und die Knochenschraube gegen den Rand des Langloches gepreßt und festgeklemmt.

Bei einer besonders bevorzugten Ausführungsform ist vorgesehen, daß die Drehachse des Exzenters und die Längsachse des Lagerkörpers zusammenfallen.

Beispielsweise kann der Lagerkörper eine fest mit dem Querträger verbundene Hülse sein, durch die hindurch ein Werkzeug zur Verdrehung des Exzenters einsetzbar ist.

Es ist dabei günstig, wenn die Hülse Aufnahmen für ein Gegenhalterwerkzeug aufweist.

Der Exzenter kann beispielsweise ein im Querträger verdrehbarer Ring sein, der am querträgerseitigen Ende der Hülse angeordnet ist. Bei einer anderen Ausführungsform ist vorgesehen, daß der Exzenter an einem in der Hülse drehbar gelagerten Kern angeordnet ist, der sich zumindest über einen Teil ihrer Länge innerhalb der Hülse erstreckt.

Bei einer anderen Ausführungsform ist der Lagerkörper im Querträger um seine Längsachse verdrehbar und trägt selbst den Exzenter. Bei einer solchen Ausgestaltung ist es in jedem Fall notwendig, die Drehverbindung zwischen Querträger und Knochenschraube durch Spannung des Exzenters festzulegen, bevor man auf dem Lagerkörper selbst den dort verdrehbaren Exzenterkörper zur Spannung der Klemmbacken in die Spannstellung verdreht.

Der Exzenter kann im Querträger in axialer Richtung festgelegt sein.

Die nachfolgende Beschreibung bevorzugter Ausführungsformen der Erfindung dient im Zusammenhang mit der Zeichnung der näheren Erläuterung. Es zeigen:
- Figur 1:: eine perspektivische Ansicht einer orthopädischen Fixationseinrichtung mit einem stabförmigen Fixierelement und einer Klemmeinrichtung zur Festlegung dieses stabförmigen Fixierelementes auf dem Schaft einer Knochenschraube;
- Figur 2:: eine Ansicht ähnlich Figur 1 mit einem Querträger zwischen Fixationseinrichtung und Knochenschraube;
- Figur 3:: eine Draufsicht auf den Klemmteil der in Figur 1 dargestellten Fixationseinrichtung mit nicht gegeneinander gespannten Klemmbacken;
- Figur 4:: eine Schnittansicht längs Linie 4-4 in Figur 3;
- Figur 5:: eine Seitenansicht der Klemmeinrichtung der Figur 4;
- Figur 6:: eine Ansicht ähnlich Figur 3 mit gespannten Klemmbacken;
- Figur 7:: eine Schnittansicht längs Linie 7-7 in Figur 6;
- Figur 8:: eine perspektivische Ansicht der Fixationseinrichtung der Figur 1 mit abgenommenen und zum Aufstecken um 90° gedrehten Klemmbacken;
- Figur 9:: eine Ansicht ähnlich Figur 4 bei einem abgewandelten Ausführungsbeispiel einer Fixationseinrichtung mit einem frei verschieblichen Zwischenstück;
- Figur 10:: eine Schnittansicht längs Linie 10-10 in Figur 9;
- Figur 11:: eine Draufsicht auf die Fixationseinrichtung der Figur 9;
- Figur 12:: eine perspektivische Ansicht eines abgewandelten Ausführungsbeispiels einer Fixationseinrichtung mit einer Schwenkachse neben der Exzenterachse;
- Figur 13:: eine schematische Schnittansicht durch ein weiteres bevorzugtes Ausführungsbeispiel einer Fixationseinrichtung mit kugeliger Verschwenkbarkeit;
- Figur 14:: eine schematische Längsschnittansicht eines weiteren bevorzugten Ausführungsbeispiels einer Fixationseinrichtung mit einem konusförmigen Anschlag für die Klemmbacken;
- Figur 15:: eine Draufsicht auf einen Querträger zur Verbindung von Fixationseinrichtung und einer Knochenschraube mit einer Exzenterklemmung im gelösten Zustand;
- Figur 16:: eine Ansicht ähnlich Figur 15 mit gespanntem Exzenter;
- Figur 17:: eine Schnittansicht längs Linie 17-17 in Figur 16;
- Figur 18:: eine Schnittansicht längs Linie 18-18 in Figur 16;
- Figur 19:: eine Ansicht ähnlich Figur 17 mit einer Lagerung an einem kugeligen Knochenschraubenkopf;
- Figur 20:: eine Ansicht ähnlich Figur 19 bei einer Lagerung an einem konischen Knochenschraubenkopf;
- Figur 21:: eine Ansicht ähnlich Figur 20 bei einem anderen bevorzugten Ausführungsbeispiel mit einem Exzenter in Form eines drehbaren Kernes und einem diesen umgebenden, mit dem Querträger fest verbundenen Lagerkörper und
- Figur 22: eine Ansicht ähnlich Figur 1 mit einem Haken statt einer Knochenschraube.

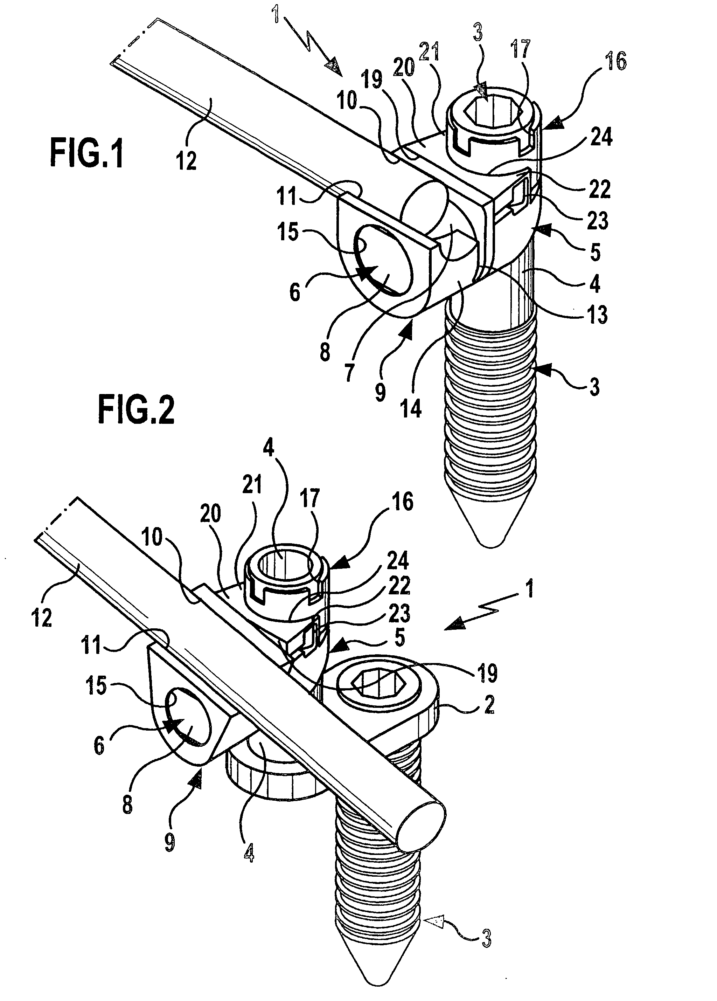

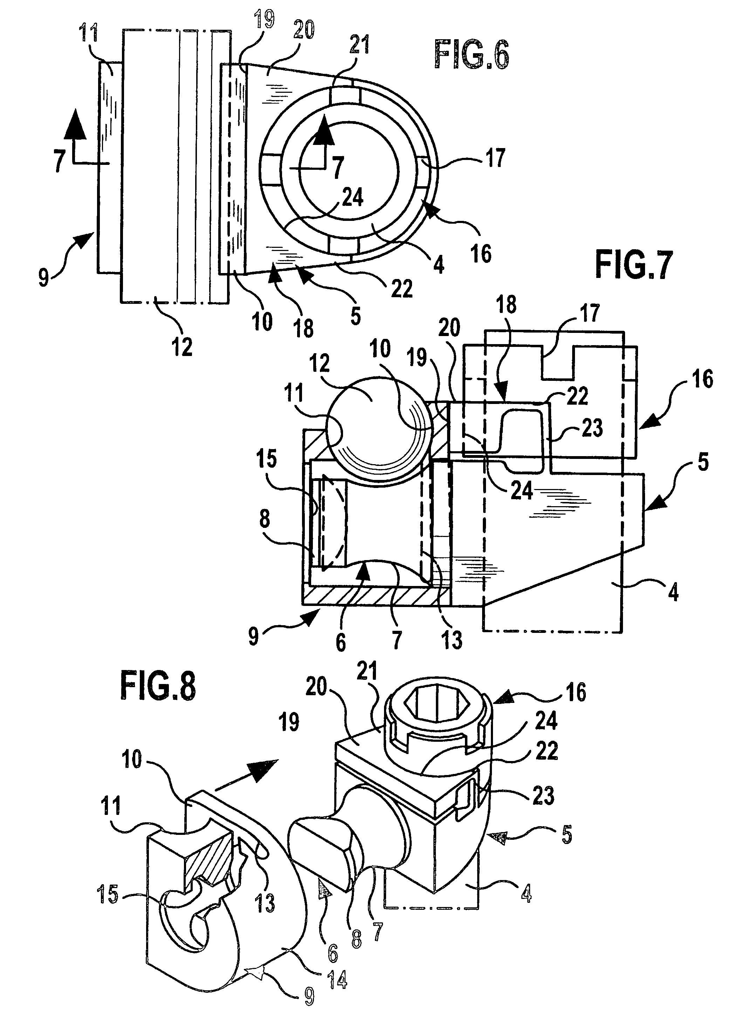

Die in den Figuren 1 bis 8 dargestellte orthopädische Fixationseinrichtung 1 ist im Ausführungsbeispiel der Figur 1 auf den Schaft einer Knochenschraube 3 aufgesetzt, im Ausführungsbeispiel der Figur 2 dagegen auf einen hülsenförmigen, zylindrischen Lagerkörper 4, der über einen Querträger 2 seitlich versetzt an der Knochenschraube 3 angeordnet ist. Im folgenden werden sowohl der Schaft der Knochenschraube 3 als auch der zylindrische Lagerkörper 4 des Querträgers 2 gemeinsam als Lagerkörper 4 bezeichnet, es versteht sich aber, daß die beschriebene orthopädische Fixationseinrichtung 1 auf die verschiedensten stift-, schaft-, oder hülsenförmigen Lagerkörper aufgesetzt werden kann.

Auf den Lagerkörper 4 ist von der Oberseite her ein Träger 5 aufgeschoben, der den Lagerkörper 4 dicht umgibt und auf diesem frei drehbar ist. Dieser Träger 5 weist eine quer zur Längsrichtung des Lagerkörpers 4 von ihm abstehende Lagerwelle 6 auf, die über ihre Länge eine Umfangsnut 7 mit bogenförmigem Querschnitt aufweist und die an ihrem freien Ende in einem auf gegenüberliegenden Seiten abgeflachten Ringflansch 8 endet.

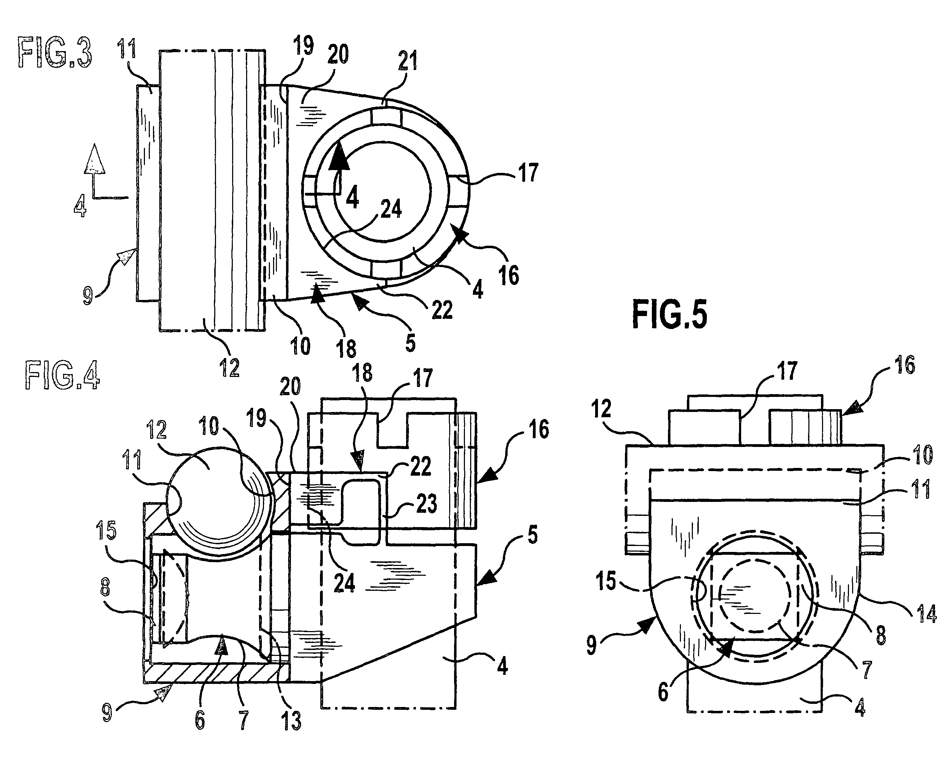

Auf diese Lagerwelle 6 ist ein im wesentlichen U-förmiger Klemmkörper 9 aufgeschoben mit zwei im wesentlichen parallel zueinander verlaufenden Seitenwänden, die zwei Klemmbacken 10, 11 ausbilden. Dazu sind die Seitenwände im Bereich oberhalb der Lagerwelle 6 auf gegenüberliegenden Seiten schalenförmig ausgebildet, so daß sie zwischen sich ein stabförmiges Fixierglied 12 aufnehmen können, welches quer zur Längsrichtung der Lagerwelle 6 angeordnet ist. Die beiden Klemmbacken 10 und 11 sind durch einen Einschnitt 13 in dem sie verbindenden Steg 14 des Klemmkörpers 9 gegeneinander federnd ausgebildet, so daß das Fixierglied 12 von oben her elastisch zwischen die beiden Klemmbacken 10, 11 eingeschnappt werden kann. In diesem eingeschnappten Zustand taucht das Fixierglied in die Umfangsnut 7 der Lagerwelle 6 ein (Figur 4).

Durch den Klemmkörper 9 führt eine durchgehende Lagerbohrung 15 hindurch, welche die Lagerwelle 6 aufnimmt und den Klemmkörper 9 dadurch um die Lagerwelle 6 verdrehbar lagert. Der Ringflansch 8 der Lagerwelle 6 liegt außenseitig an dem Klemmkörper 9 an und verhindert somit ein Abziehen des Klemmkörpers 9 von der Lagerwelle 6.

Die Lagerbohrung 15 ist so ausgebildet, daß bei einer Verdrehung des Klemmkörpers 9 gegenüber dem Träger 5 um 90° ein Abziehen des Klemmkörpers 9 von der Lagerwelle 6 vorgenommen werden kann, dies wird durch die seitlichen Abflachungen am Ringflansch 8 der Lagerwelle 6 ermöglicht. Man erhält somit eine bajonettartige Verriegelung; durch eine Verformung des Ringflansches kann nach dem Aufsetzen des Klemmkörpers 9 eine dauerhafte, ein Abziehen verhindernde Festlegung des Klemmkörpers 9 auf der Lagerwelle 6 erreicht werden.

Auf dem Lagerkörper 4 ist oberhalb des Trägers 5 ein den Lagerkörper 4 umgebender Ring drehbar gelagert, dessen Außenumfang gegenüber der durch den Lagerkörper 4 definierten Drehachse exzentrisch ist, dieser Ring bildet somit einen Exzenterkörper 16 aus. Dieser Exzenterkörper 16 ist an seinem oberen Rand mit axialen Einschnitten 17 versehen, in welche ein in der Zeichnung nicht dargestelltes Drehwerkzeug eingreifen kann, mit dessen Hilfe der Exzenterkörper 16 um den Lagerkörper 4 verdreht werden kann.

Zwischen dem Exzenterkörper 16 und dem angrenzenden Klemmbacken 10 ist ein im wesentlichen U-förmiges Zwischenstück 18 eingesetzt, welches sich mit der Außenseite 19 seines Verbindungssteges 20 an dem Klemmbacken 10 abstützt, während die beiden an den Verbindungssteg 20 anschließenden Schenkel 21, 22 an ihrem freien Ende in parallel zum Lagerkörper 4 verlaufende Federstege 23 übergehen, die das Zwischenstück 18 mit dem Träger 5 verbinden. Diese Federstege 23 ermöglichen eine geringfügige federnde Verschiebung des Zwischenstückes 18 in Richtung auf die Klemmbacken 10 und 11.

Die Schenkel 21 und 22 bilden eine im wesentlichen halbkreisförmige innere Anlagefläche 24 aus, welche den Exzenterkörper 16 umgibt.

In einer Lösestellung steht der Exzenterkörper 16 am wenigsten weit in Richtung auf die Klemmbacken 10, 11 vor. Wird der Exzenterkörper 16 jedoch verdreht, schiebt sich sein Umfang zunehmend in Richtung auf die Klemmbacken 10, 11 vor und verschiebt dabei das Zwischenstück 18 in Richtung auf die Klemmbacken 10, 11. Dadurch wird der Klemmbacken 10, der im folgenden als beweglicher Klemmbacken bezeichnet wird, in Richtung auf den gegenüberliegenden Klemmbacken 11 verschoben, der im folgenden als fester Klemmbacken bezeichnet wird. Der bewegliche Klemmbacken 10 legt sich an das stabförmige Fixierglied 12 an und verschiebt dieses in Richtung auf den festen Klemmbacken 11, dadurch wird gleichzeitig der Klemmkörper 9 gegen den Ringflansch 8 gedrückt. Der Exzenterkörper 16 stützt sich dabei an dem Lagerkörper 4 ab, und als Ergebnis dieser Spannung des Exzenterkörpers 16 werden alle Freiheitsgrade der beschriebenen Vorrichtung durch Klemmung verriegelt. Der Klemmkörper 9 wird durch die kräftige Anlage an dem Ringflansch 8 gegenüber einer Drehung um die Achse der Lagerwelle 6 fixiert, das Fixierglied 12 wird zwischen den beiden gegeneinander gespannten Klemmbacken 10, 11 festgelegt, und der Träger 5 wird durch die Anlage des Exzenterkörpers 16 am Lagerkörper 4 gegen eine Drehung um den Lagerkörper 4 gesichert. Man erhält also allein durch Verdrehung des Exzenterkörpers 16 eine Festlegung von vier unterschiedlichen Freiheitsgraden der beschriebenen Anordnung. Dabei ist von Bedeutung, daß diese Festlegung von oben her erfolgen kann, ebenso wie das Fixierglied 12 von oben her zwischen die noch nicht gespannten Klemmbacken 10, 11 schnappend eingeführt werden kann. Der Operateur hat also die Möglichkeit, alle wesentlichen Schritte zum Zusammenbau und zum Fixieren der Fixationseinrichtung 1 durch eine sehr kleine Zugangsöffnung von oben her vorzunehmen.

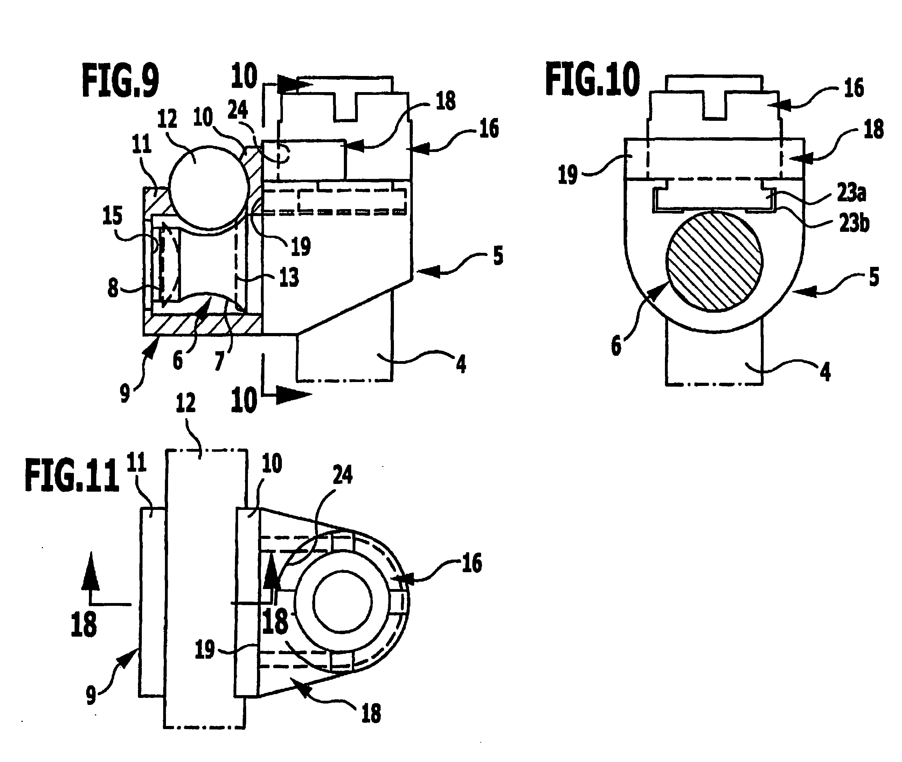

Bei dem Ausführungsbeispiel der Figuren 9 bis 11 ist ein ähnlicher Aufbau gewählt. Einander entsprechende Teile tragen daher dieselben Bezugszeichen. Im Unterschied zum Ausführungsbeispiel der Figuren 1 bis 8 ist hier das Zwischenstück 18 nicht über Federstege mit dem Träger 5 verbunden, sondern von diesem vollständig getrennt. Allerdings greift das Zwischenstück 18 mit einer flanschförmigen Verbreiterung 23a in eine parallel zur Klemmrichtung der Klemmbacken 10, 11 verlaufende Führungsnut 23b ein, so daß das Zwischenstück 18 im Träger 5 längsverschieblich geführt ist. Auf diese Weise können auch größere Verschiebungen des Zwischenstückes realisiert werden, die möglicherweise zu einer zu starken Verbiegung der Federstege 23 führen könnten.

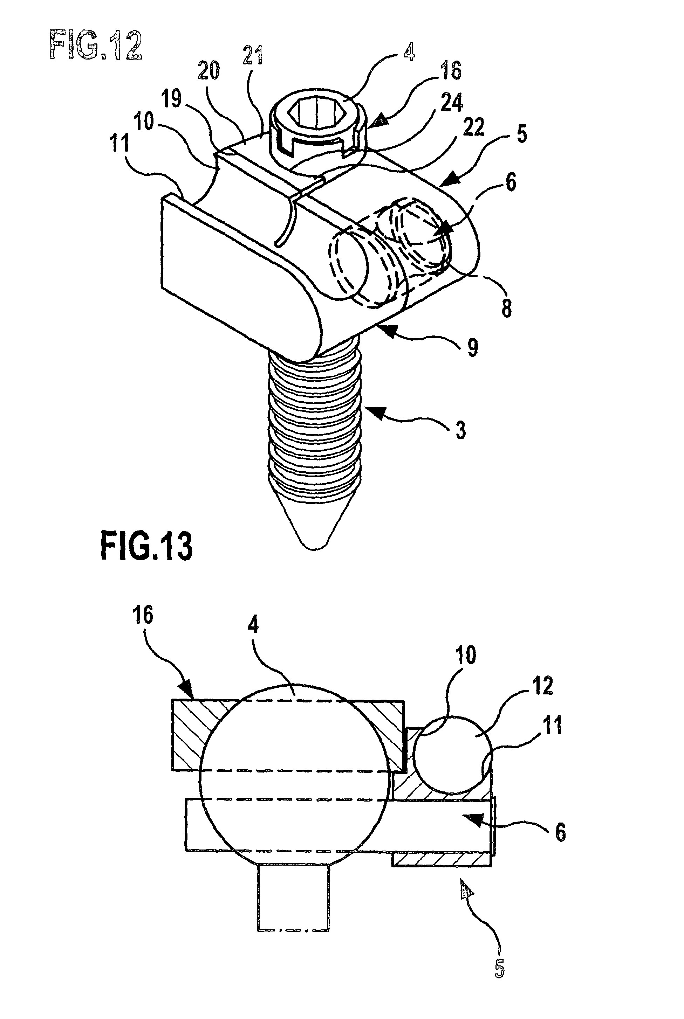

Bei dem Ausführungsbeispiel der Figur 12 ist ein ähnlicher Aufbau gewählt wie bei dem Ausführungsbeispiel der Figur 1, die Lagerwelle 6 ist jedoch hier seitlich neben die Knochenschraube 3, den Exzenterkörper 16 und das Zwischenstück 18 verlagert worden, bei dem dargestellten Ausführungsbeispiel ist außerdem die Lagerwelle 6 nicht am Träger 5 angeordnet, sondern am Klemmkörper 9 und ragt in eine entsprechende Aufnahmebohrung in den Träger 5 hinein. Dies sind Abwandlungen, die einzeln oder gemeinsam vorgenommen werden können und zu geänderten Geometrien der Gesamtanordnung führen, am Prinzip jedoch nichts ändern.

Bei dem Ausführungsbeispiel der Figur 13, das dort nur sehr schematisch wiedergegeben ist und bei dem Teile, die den bisher beschriebenen Ausführungen entsprechen, dieselben Bezugszeichen tragen, ist ein kugeliger Lagerkörper 4 verwendet, beispielsweise ein Kugelkopf an einer Knochenschraube. Damit ist die gesamte Fixationseinrichtung nicht nur um eine Achse verdrehbar an der Knochenschraube gelagert, sondern kann zusätzlich auch noch in andere Richtungen verschwenkt und dann durch den Exzenterkörper 16 an dem kugelkopfförmigen Lagerkörper 4 festgeklemmt werden.

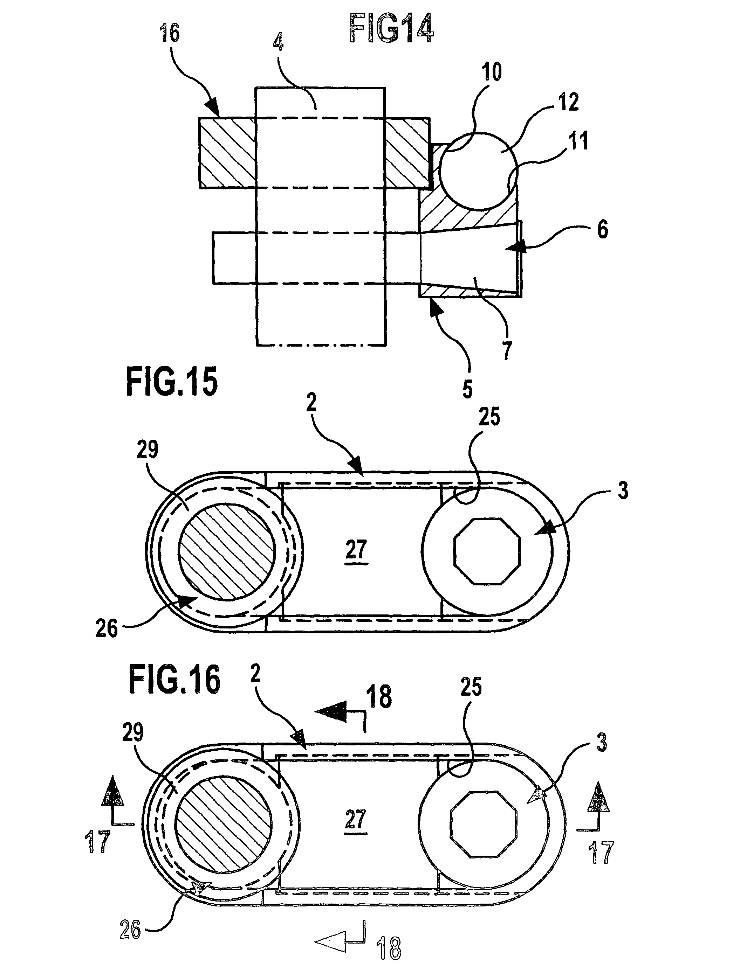

Beim Ausführungsbeispiel der Figur 14, bei dem wieder einander entsprechende Teile dieselben Bezugszeichen tragen, ist die Lagerwelle 6 konisch ausgebildet, so daß der Klemmkörper 9 beim Spannen des Exzenterkörpers 16 gegen das konisch dicker werdende Ende der Lagerwelle 6 gedrückt und damit festgelegt wird.

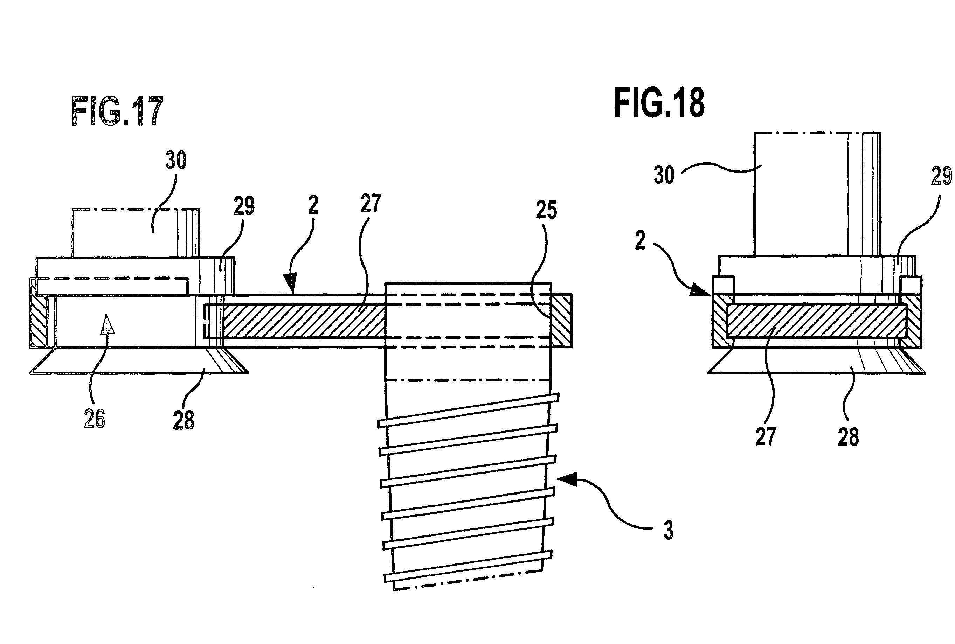

Bei dem Ausführungsbeispiel der Figur 2 ist die Fixationseinrichtung über einen Querträger 2 an der Knochenschraube 3 gehalten. Bei einem bevorzugten Ausführungsbeispiel eines solchen Querträgers 2, wie er beispielsweise in den Figuren 15 bis 18 dargestellt ist, ist dieser Querträger 2 als flache, streifenförmige Platte ausgebildet und trägt ein sich über einen großen Teil seiner Länge erstreckendes Langloch 25. Durch dieses Langloch ist die Knochenschraube 3 hindurchgesteckt, außerdem ist in diesem Langloch 25 auf der gegenüberliegenden Seite desselben ein Exzenter 26 drehbar gelagert. Zwischen der Knochenschraube 3 und dem Exzenter 26 ist längsverschieblich in dem Langloch 25 ein Klemmstück 27 angeordnet, welches sich sowohl an der Knochenschraube 3 als auch am Exzenter 26 abstützt.

In der Darstellung der Figuren 15 bis 18 ist der Lagerkörper 4 der besseren Übersichtlichkeit halber nicht dargestellt, dieser Lagerkörper könnte beispielsweise den Exzenter 26 umgebend mit dem Querträger 2 verschweißt sein.

In der gelösten Stellung hat der Umfang des Exzenters 26 von der Knochenschraube 3 einen maximalen Abstand, beim Verdrehen des Exzenters 26 wird dieser Abstand kleiner, so daß dadurch das Klemmstück 27 in Richtung auf die Knochenschraube 3 verschoben wird und schließlich diese gegen den Rand des Langloches 25 spannt und dadurch den Querträger 2 fest mit der Knochenschraube 3 verbindet. Bei gelöstem Exzenter 26 kann der Operateur also den Querträger 2 auf der Knochenschraube 3 in die gewünschte Stellung verdrehen, diese Stellung kann allein durch Spannen des Exzenters 26 fixiert werden.

Es ergibt sich damit eine zusätzliche Möglichkeit, die orthopädische Fixationseinrichtung, die in der Figur 2 dargestellt ist, relativ zur Knochenschraube 3 in unterschiedliche Positionen zu bringen, so daß eine besonders große Variationsmöglichkeit für die Anlegepositionen erreicht wird. Auch in diesem Falle erfolgt die Betätigung des Exzenters 26 an derselben Stelle wie die Betätigung des Exzenterkörpers 16, da beide koaxial zueinander angeordnet sind, so daß auch hier die Feststellung der Bewegungsmöglichkeiten durch dieselbe sehr kleine Zugangsöffnung möglich ist.

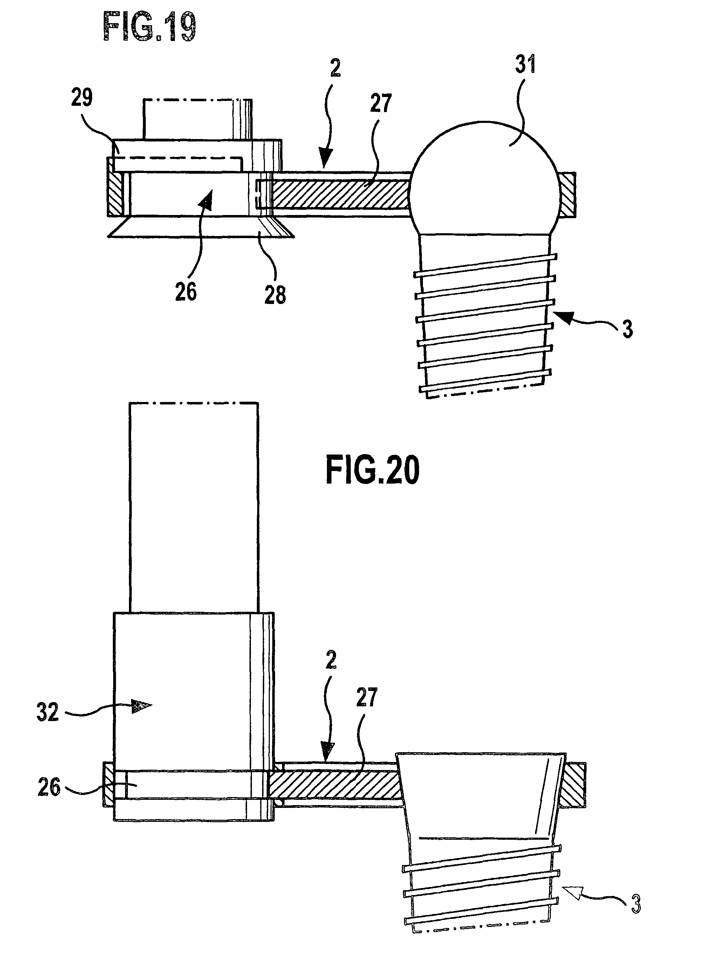

Bei dem Ausführungsbeispiel der Figuren 15 bis 18 hat die Knochenschraube 3 im Bereich des Querträgers 2 einen zylindrischen Außenschaft, bei dem Ausführungsbeispiel der Figur 19, das sonst gleich aufgebaut ist, ist die Knochenschraube 3 in diesem Bereich kugelig ausgebildet und wird zwischen dem Klemmstück 27 einerseits und dem Querträger 2 andererseits axial unverschieblich, jedoch frei verschwenkbar, gehalten. Wird das Klemmstück 27 beim Spannen des Exzenters 26 gegen diesen kugeligen Lagerbereich 31 verschoben, so wird auch die unterschiedliche Schwenkrichtung der Knochenschraube 3 im Querträger 2 dauerhaft fixiert.

Das Ausführungsbeispiel der Figur 20 entspricht weitgehend dem der Figuren 15 bis 18, einander entsprechende Teile tragen daher dieselben Bezugszeichen. Während der Exzenter 26 beim Ausführungsbeispiel der Figuren 15 bis 18 ringförmig ausgebildet ist, wird bei dem Ausführungsbeispiel der Figur 20 ein Exzenter 16 in Form eines drehbaren Kernes 32 verwendet, der in seinem unteren, im Querträger 2 angeordneten Bereich den Exzenter 26 ausbildet. Der Kern 32 kann durch ein Werkzeug verdreht werden, das von oben formschlüssig mit diesem verbunden wird.

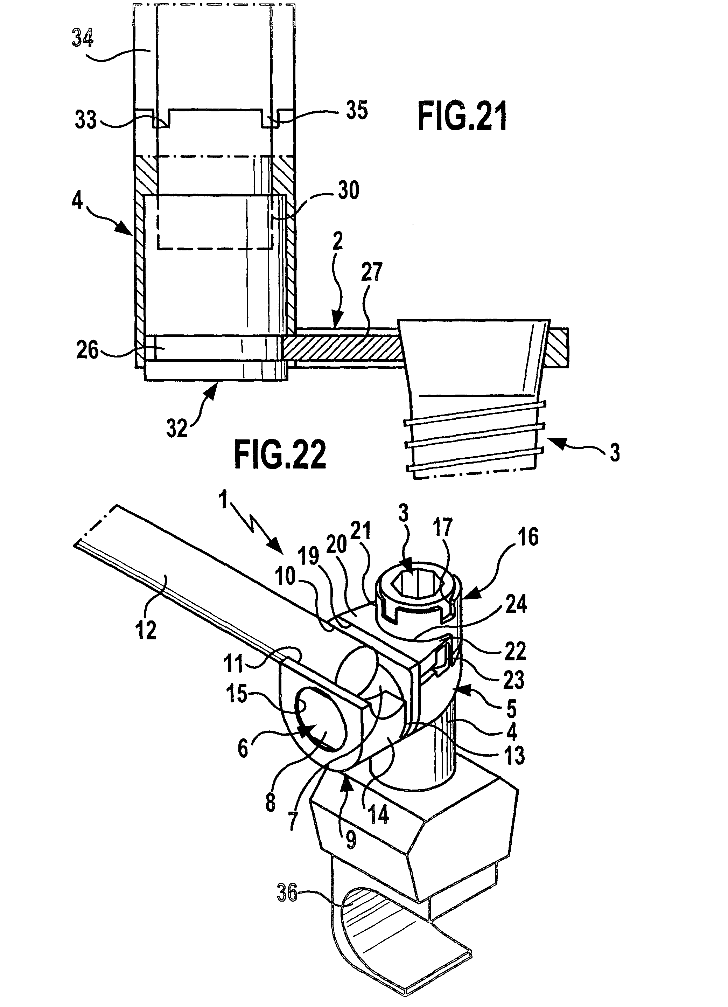

Der Kern 32 kann selbst den Lagerkörper 4 ausbilden, an dem die Fixationseinrichtung 1 festgelegt wird, es kann aber auch vorgesehen sein, daß der Lagerkörper 4 als Hülse ausgebildet ist, die fest mit dem Querträger 2 verbunden wird, wie dies bei dem Ausführungsbeispiel der Figur 21 dargestellt ist, welches sonst dem Ausführungsbeispiel der Figur 20 entspricht. Zu Verdrehung des Kernes 32 kann mit einem Drehwerkzeug 30 in das Innere des hohlen Lagerkörpers 4 eingegriffen werden. Günstig ist es außerdem, wenn zusätzlich ein Gegenhalterwerkzeug 34 vorgesehen wird, beispielsweise in Form einer auf den Lagerkörper 4 aufsetzbaren Hülse, welches mit Vorsprüngen 35 in entsprechende Ausnehmungen 33 des Lagerkörpers 4 formschlüssig eingreift. Auf diese Weise ist es möglich, ohne Übertragung von Drehmomenten auf den Querträger 2 den Kern 32 gegenüber dem Lagerkörper 4 zu verdrehen und dadurch den Exzenter 26 in die Spannstellung zu bringen.

Die Knochenschraube 3 der vorstehend beschriebenen Ausführungsbeispiele kann auch ersetzt werden durch einen Haken 36, der eine knöcherne Struktur umgreift. Dieser Haken kann mittels eines schaftförmigen Oberteils ebenso einen Querträger 2 oder einen Träger 5 tragen wie die Knochenschraube, lediglich die Verbindung zum Knochen erfolgt nicht über ein Gewinde, sondern über ein hakenförmiges Ende. Dieser Austausch ist bei allen vorstehend beschriebenen Ausführungsbeispielen möglich.

## Patentansprüche

1. Orthopädische Fixationseinrichtung (1) zur Festlegung eines stabförmigen Fixierelementes (12) mit zwei gegeneinander bewegbaren, bei Annäherung das Fixierelement (12) zwischen sich einspannenden Klemmbacken (10, 11), **dadurch gekennzeichnet, daß** in Spannrichtung neben den Klemmbacken (10, 11) ein Exzenterkörper (16) drehbar an der Fixationseinrichtung (1) gelagert ist, der beim Verdrehen einen Klemmbacken (10) gegen den anderen Klemmbacken (11) verschiebt.

2. Einrichtung nach Anspruch 1, **dadurch gekennzeichnet, daß** der Exzenterkörper (16) auf einem rotationssymmetrischen Lagerkörper (4) drehbar gelagert ist, an dem auch die Klemmbacken (10, 11) gehalten sind.

3. Einrichtung nach Anspruch 2, **dadurch gekennzeichnet, daß** die Drehachse des Exzenterkörpers (16) parallel zur Achse des rotationssymmetrischen Lagerkörpers (4) verläuft.

4. Einrichtung nach Anspruch 3, **dadurch gekennzeichnet, daß** die Drehachse des Exzenterkörpers (16) mit der Achse des rotationssymmetrischen Lagerkörpers (4) identisch ist.

5. Einrichtung nach einem der Ansprüche 2 bis 4, **dadurch gekennzeichnet, daß** der rotationssymmetrische Lagerkörper (4) kreiszylindrisch ausgebildet ist.

6. Einrichtung nach einem der Ansprüche 2 bis 4, **dadurch gekennzeichnet, daß** der rotationssymmetrische Lagerkörper (4) kugelig ausgebildet ist.

7. Einrichtung nach einem der Ansprüche 2 bis 4, **dadurch gekennzeichnet, daß** der rotationssymmetrische Lagerkörper konisch ausgebildet ist.

8. Einrichtung nach einem der Ansprüche 2 bis 7, **dadurch gekennzeichnet, daß** an dem rotationssymmetrischen Lagerkörper (4) ein Träger (5) gehalten ist, der die beiden Klemmbacken (10, 11) trägt.

9. Einrichtung nach Anspruch 8, **dadurch gekennzeichnet, daß** der Träger (5) starr mit dem Lagerkörper (4) verbunden ist.

10. Einrichtung nach Anspruch 8, **dadurch gekennzeichnet, daß** der Träger (5) um die Symmetrieachse des rotationssymmetrischen Lagerkörpers (4) verdrehbar an diesem gehalten ist.

11. Einrichtung nach Anspruch 10, **dadurch gekennzeichnet, daß** der Träger (5) in Richtung der Drehachse auf den rotationssymmetrischen Lagerkörper (4) aufschiebbar ist.

12. Einrichtung nach einem der voranstehenden Ansprüche, **dadurch gekennzeichnet, daß** der eine der beiden Klemmbacken als fester Klemmbacken (11) ausgebildet ist, dessen Abstand von der Drehachse des Exzenterkörpers (16) im wesentlichen fest ist, während der andere als beweglicher Klemmbacken (10) ausgebildet ist, der durch den Exzenterkörper (16) gegen den festen Klemmbacken (11) bewegbar ist.

13. Einrichtung nach einem der Ansprüche 8 bis 12, **dadurch gekennzeichnet, daß** der feste Klemmbacken (11) starr mit dem Träger (5) verbunden ist.

14. Einrichtung nach einem der Ansprüche 1 bis 12, **dadurch gekennzeichnet, daß** am Träger (5) ein Anschlag (8) für den festen Klemmbacken (11) angeordnet ist, der eine Verschiebung des festen Klemmbackens (11) in Klemmrichtung der Klemmbacken (10, 11) und damit eine Entfernung des festen Klemmbackens (11) von der Drehachse des Exzenterkörpers (16) begrenzt.

15. Einrichtung nach einem der Ansprüche 8 bis 12 oder 14, **dadurch gekennzeichnet, daß** die Klemmbacken (10, 11) um eine parallel zur Klemmrichtung der Klemmbacken (10, 11) verlaufende Drehachse verschwenkbar mit dem Träger (5) verbunden sind.

16. Einrichtung nach Anspruch 15, **dadurch gekennzeichnet, daß** die Drehachse der Klemmbacken (10, 11) und die Drehachse des Exzenterkörpers (16) in einer Ebene liegen.

17. Einrichtung nach Anspruch 15, **dadurch gekennzeichnet, daß** die Drehachse der Klemmbacken (10, 11) und die Drehachse des Exzenterkörpers (16) seitlich gegeneinander versetzt sind.

18. Einrichtung nach einem der Ansprüche 15 bis 17, **dadurch gekennzeichnet, daß** beide Klemmbacken (10, 11) durch die Seitenwände eines im Querschnitt U-förmigen Klemmkörpers (9) gebildet werden, dessen Seitenwände federnd gegeneinander biegbar sind.

19. Einrichtung nach einem der Ansprüche 15 bis 18, **dadurch gekennzeichnet, daß** die Schwenkverbindung zwischen Klemmbacken (10, 11) und Träger (5) lösbar ist.

20. Einrichtung nach Anspruch 19, **dadurch gekennzeichnet, daß** die Schwenkverbindung zwei bajonettartig einander hintergreifende Lagerglieder umfaßt, die sich in einer bestimmten Winkelstellung nicht hintergreifen und dann axial gegeneinander verschiebbar sind.

21. Einrichtung nach einem der voranstehenden Ansprüche, **dadurch gekennzeichnet, daß** zwischen dem Exzenterkörper (16) und den Klemmbacken (10, 11) ein Zwischenglied (18) angeordnet ist, welches beim Verdrehen des Exzenterkörpers (16) gegen die Klemmbacken (10, 11) verschoben wird.

22. Einrichtung nach Anspruch 21, **dadurch gekennzeichnet, daß** das Zwischenglied (18) beweglich an dem Träger (5) gehalten ist.

23. Einrichtung nach Anspruch 22, **dadurch gekennzeichnet, daß** das Zwischenglied (18) über eine federnde Verbindung (23) an dem Träger (5) gehalten ist.

24. Einrichtung nach einem der voranstehenden Ansprüche, **dadurch gekennzeichnet, daß** die Klemmbacken (10, 11) bei gelöstem Exzenterkörper (16) federnd so weit auseinander bewegbar sind, daß ein stabförmiges Fixierelement (12) zwischen sie einschnappbar ist.

25. Einrichtung nach einem der Ansprüche 2 bis 24, **dadurch gekennzeichnet, daß** der Exzenterkörper (16) als Ring ausgebildet ist.

26. Einrichtung nach einem der voranstehenden Ansprüche, **dadurch gekennzeichnet, daß** der Exzenterkörper (16) Ausnehmungen (17) zur Aufnahme eines Drehwerkzeuges aufweist.

27. Einrichtung nach einem der Ansprüche 2 bis 26, **dadurch gekennzeichnet, daß** der Lagerkörper (4) Teil einer Knochenschraube (3) ist.

28. Einrichtung nach einem der Ansprüche 2 bis 26, **dadurch gekennzeichnet, daß** der Lagerkörper (4) Teil eines Hakens (36) ist.

29. Einrichtung nach einem der Ansprüche 2 bis 26, **dadurch gekennzeichnet, daß** der Lagerkörper (4) über einen Querträger (2) mit einer Knochenschraube (3) oder einem Haken (36) verbunden ist und daß die Längsachse der Knochenschraube (3) oder des Hakens (36) seitlich versetzt zur Längsachse des Lagerkörpers (4) verläuft.

30. Einrichtung nach Anspruch 29, **dadurch gekennzeichnet, daß** die Knochenschraube (3) oder der Haken (36) im Querträger (2) um ihre Längsachse verdrehbar und in einer bestimmten Winkelstellung festklemmbar sind.

31. Einrichtung nach Anspruch 30, **dadurch gekennzeichnet, daß** zur Festklemmung im Querträger (2) ein Klemmstück (27) gegen die Knochenschraube (3) oder den Haken (36) verschiebbar ist.

32. Einrichtung nach Anspruch 31, **dadurch gekennzeichnet, daß** zur Verschiebung des Klemmstückes (27) im Querträger (2) ein am Klemmstück (27) anliegender, um eine Drehachse verdrehbarer Exzenter (26) gelagert ist.

33. Einrichtung nach Anspruch 32, **dadurch gekennzeichnet, daß** die Drehachse des Exzenters (26) senkrecht auf der Ebene des Querträgers (2) steht.

34. Einrichtung nach Anspruch 33, **dadurch gekennzeichnet, daß** die Drehachse des Exzenters (26) parallel zur Längsachse der Knochenschraube (3) oder des Hakens (36) verläuft.

35. Einrichtung nach einem der Ansprüche 32 bis 34, **dadurch gekennzeichnet, daß** die Drehachsen des Exzenters (26) im Querträger (2) und des Exzenterkörpers (16) parallel zueinander verlaufen.

36. Einrichtung nach Anspruch 35, **dadurch gekennzeichnet, daß** die Drehachsen des Exzenters (26) und des Exzenterkörpers (16) zusammenfallen.

37. Einrichtung nach Anspruch 33, **dadurch gekennzeichnet, daß** die Knochenschraube (3) oder der Haken (36) durch eine kugelige Verbindung mit dem Querträger (2) verschwenkbar verbunden ist und das Klemmstück (27) beim Fixieren in Richtung auf eine kugelige Lagerfläche (31) der Knochenschraube (3) bzw. des Hakens (36) verschiebbar ist.

38. Einrichtung nach einem der Ansprüche 31 bis 37, **dadurch gekennzeichnet, daß** der Querträger (2) ein Langloch (25) aufweist, in welchem die Knochenschraube (3) oder der Haken (36), das Klemmstück (27) und der Exzenter (26) aufgenommen sind.

39. Einrichtung nach einem der Ansprüche 32 bis 38, **dadurch gekennzeichnet, daß** die Drehachse des Exzenters (26) und die Längsachse des Lagerkörpers (4) zusammenfallen.

40. Einrichtung nach Anspruch 39, **dadurch gekennzeichnet, daß** der Lagerkörper (4) eine fest mit dem Querträger (2) verbundene Hülse ist, durch die hindurch ein Werkzeug (30) zur Verdrehung des Exzenters (26) einsetzbar ist.

41. Einrichtung nach Anspruch 40, **dadurch gekennzeichnet, daß** der Lagerkörper (4) Aufnahmen (33) für ein Gegenhalterwerkzeug (34) aufweist.

42. Einrichtung nach Anspruch 40 oder 41, **dadurch gekennzeichnet, daß** der Exzenter (26) ein im Querträger (2) verdrehbarer Ring ist, der am querträgerseitigen Ende des Lagerkörpers (4) angeordnet ist.

43. Einrichtung nach Anspruch 40 oder 41, **dadurch gekennzeichnet, daß** der Exzenter (26) an einem im Lagerkörper (4) drehbar gelagerten Kern (32) angeordnet ist, der sich zumindest über einen Teil seiner Länge innerhalb des Lagerkörpers (4) erstreckt.

44. Einrichtung nach Anspruch 39, **dadurch gekennzeichnet, daß** der Lagerkörper (4) im Querträger (2) um seine Längsachse verdrehbar ist und selbst den Exzenter (26) trägt.

45. Einrichtung nach einem der Ansprüche 32 bis 44, **dadurch gekennzeichnet, daß** der Exzenter (26) im Querträger (2) in axialer Richtung festgelegt ist.

## Claims

1. Orthopedic fixation device (1) for securing a rod-like fixation element (12), with two clamping jaws (10, 11) which can be moved relative to one another and which, when brought together, clamp the fixation element (12) between them, **characterized by** a cam body (16) which is mounted on the fixation device (1) so as to be able to rotate next to the clamping jaws (10, 11) in the direction of tightening and which, when rotated, pushes one clamping jaw (10) towards the other clamping jaw (11).

2. Device according to Claim 1, **characterized in that** the cam body (16) is mounted rotatably on a rotationally symmetrical mounting body (4) on which the clamping jaws (10, 11) are also held.

3. Device according to Claim 2, **characterized in that** the rotation axis of the cam body (16) extends parallel to the axis of the rotationally symmetrical mounting body (4).

4. Device according to Claim 3, **characterized in that** the rotation axis of the cam body (16) is identical to the axis of the rotationally symmetrical mounting body (4).

5. Device according to one of Claims 2 to 4, **characterized in that** the rotationally symmetrical mounting body (4) has a circular cylinder configuration.

6. Device according to one of Claims 2 to 4, **characterized in that** the rotationally symmetrical mounting body (4) has a spherical configuration.

7. Device according to one of Claims 2 to 4, **characterized in that** the rotationally symmetrical mounting body has a conical configuration.

8. Device according to one of Claims 2 to 7, **characterized in that** a support piece (5), which carries the two clamping jaws (10, 11), is held on the rotationally symmetrical mounting body (4).

9. Device according to Claim 8, **characterized in that** the support piece (5) is rigidly connected to the mounting body (4).

10. Device according to Claim 8, **characterized in that** the support piece (5) is held on the rotationally symmetrical mounting body (4) so as to be rotatable about the axis of symmetry of said mounting body.

11. Device according to Claim 10, **characterized in that** the support piece (5) can be pushed onto the rotationally symmetrical mounting body (4) in the direction of the rotation axis.

12. Device according to one of the preceding claims, **characterized in that** one of the two clamping jaws is configured as a stationary clamping jaw (11) whose spacing from the rotation axis of the cam body (16) is substantially fixed, whereas the other one is configured as a movable clamping jaw (10) which can be moved by the cam body (16) towards the stationary clamping jaw (11).

13. Device according to one of Claims 8 to 12, **characterized in that** the stationary clamping jaw (11) is connected rigidly to the support piece (5).

14. Device according to one of Claims 1 to 12, **characterized in that** a limit stop (8) for the stationary clamping jaw (11) is disposed on the support piece (5) and limits a movement of the stationary clamping jaw (11) in the clamping direction of the clamping jaws (10, 11) and thus limits a movement of the stationary clamping jaw (11) away from the rotation axis of the cam body (16).

15. Device according to one of Claims 8 to 12 or 14, **characterized in that** the clamping jaws (10, 11) are connected to the support piece (5) so as to be able to pivot about a rotation axis extending parallel to the clamping direction of the clamping jaws (10, 11).

16. Device according to Claim 15, **characterized in that** the rotation axis of the clamping jaws (10, 11) and the rotation axis of the cam body (16) are located in one plane.

17. Device according to Claim 15, **characterized in that** the rotation axis of the clamping jaws (10, 11) and the rotation axis of the cam body (16) are laterally offset from one another.

18. Device according to one of Claims 15 to 17, **characterized in that** both clamping jaws (10, 11) are formed by the side walls of a clamping body (9) which has a U-shaped cross-section and whose side walls can be bent resiliently relative to one another.

19. Device according to one of Claims 15 to 18, **characterized in that** the pivot connection between clamping jaws (10, 11) and support piece (5) is releasable.

20. Device according to Claim 19, **characterized in that** the pivot connection comprises two mounting members which engage behind one another in a bayonet fashion and which, in a defined angular position, do not engage behind one another and are then axially movable relative to one another.

21. Device according to one of the preceding claims, **characterized in that** between the cam body (16) and the clamping jaws (10, 11) there is an intermediate member (18) which, when the cam body (16) is rotated, is moved against the clamping jaws (10, 11).

22. Device according to Claim 21, **characterized in that** the intermediate member (18) is held movably on the support piece (15).

23. Device according to Claim 22, **characterized in that** the intermediate member (18) is held on the support piece (5) via a resilient connection (23).

24. Device according to one of the preceding claims, **characterized in that**, when the cam body (16) is released, the clamping jaws (10, 11) can be moved so far apart from one another that a rod-like fixation element (12) can be snap-fitted in between them.

25. Device according to one of Claims 2 to 24, **characterized in that** the cam body (16) is formed as a ring.

26. Device according to one of the preceding claims, **characterized in that** the cam body (16) has recesses (17) for accommodating a rotary tool.

27. Device according to one of Claims 2 to 26, **characterized in that** the mounting body (4) is part of a bone screw (3).

28. Device according to one of Claims 2 to 26, **characterized in that** the mounting body (4) is part of a hook (36).

29. Device according to one of Claims 2 to 26, **characterized in that** the mounting body (4) is connected to a bone screw (3) or a hook (36) via a transverse support (2), and **in that** the longitudinal axis of the bone screw (3) or of the hook (36) extends laterally offset from the longitudinal axis of the mounting body (4).

30. Device according to Claim 29, **characterized in that** the bone screw (3) or the hook (36) is rotatable about its longitudinal axis in the transverse support (2) and can be clamped in a defined angular position.

31. Device according to Claim 30, **characterized in that**, in order to permit clamping in the transverse support (2), a clamp piece (27) can be moved against the bone screw (3) or the hook (36).

32. Device according to Claim 31, **characterized in that**, in order to move the clamp piece (27) in the transverse support (2), a cam (26) is mounted so that it engages against the clamp piece (27) and is rotatable about a rotation axis.

33. Device according to Claim 32, **characterized in that** the rotation axis of the cam (26) is perpendicular to the plane of the transverse support (2).

34. Device according to Claim 33, **characterized in that** the rotation axis of the cam (26) extends parallel to the longitudinal axis of the bone screw (3) or of the hook (36).

35. Device according to one of Claims 32 to 34, **characterized in that** the rotation axes of the cam (26) in the transverse support (2) and of the cam body (16) extend parallel to one another.

36. Device according to Claim 35, **characterized in that** the rotation axes of the cam (26) and of the cam body (16) coincide.

37. Device according to Claim 33, **characterized in that** the bone screw (3) or the hook (36) is connected pivotably to the transverse support (2) via a spherical connection, and the clamp piece (27) can be moved in the direction of a spherical engagement surface (31) of the bone screw (3) or of the hook (36) at the time of fixing.

38. Device according to one of Claims 31 to 37, **characterized in that** the transverse support (2) has an elongate hole (25) in which the bone screw (3) or the hook (36), the clamp piece (27) and the cam (26) are accommodated.

39. Device according to one of Claims 32 to 38, **characterized in that** the rotation axis of the cam (26) and the longitudinal axis of the mounting body (4) coincide.

40. Device according to Claim 39, **characterized in that** the mounting body (4) is a sleeve which is connected fixedly to the transverse support (2) and through which a tool (30) can be inserted for turning the cam (26).

41. Device according to Claim 40, **characterized in that** the mounting body (4) has seats (33) for a stabilizing tool (34).

42. Device according to Claim 40 or 41, **characterized in that** the cam (26) is a ring which is rotatable in the transverse support (2) and which is disposed at the transverse support end of the mounting body (4).

43. Device according to Claim 40 or 41, **characterized in that** the cam (26) is disposed on a core (32) which is mounted rotatably in the mounting body (4) and which, at least along part of its length, extends inside the mounting body (4).

44. Device according to Claim 39, **characterized in that** the mounting body (4) is rotatable about its longitudinal axis in the transverse support (2) and itself carries the cam (26).

45. Device according to one of Claims 32 to 44, **characterized in that** the cam (26) is secured in the axial direction in the transverse support (2).

## Revendications

1. Dispositif de fixation orthopédique (1) pour l'immobilisation d'un élément de fixation (12) en forme de barre, comprenant deux mors de serrage (10, 11) qui peuvent être déplacés l'un par rapport à l'autre et qui, en cas de rapprochement, serrent entre eux l'élément de fixation (12), **caractérisé en ce que** dans la direction de serrage, à côté des mors de serrage (10, 11), un corps d'excentrique (16) est monté rotatif sur le dispositif de fixation (1), et produit, lors de sa rotation, le déplacement d'un mors de serrage (10) vers l'autre mors de serrage (11).

2. Dispositif selon la revendication 1, **caractérisé en ce que** le corps d'excentrique (16) est monté rotatif sur un corps de palier (4) à symétrie de rotation, sur lequel sont également maintenus les mors de serrage (10, 11).

3. Dispositif selon la revendication 2, **caractérisé en ce que** l'axe de rotation du corps d'excentrique (16) s'étend parallèlement à l'axe du corps de palier (4) à symétrie de rotation.

4. Dispositif selon la revendication 3, **caractérisé en ce que** l'axe de rotation du corps d'excentrique (16) est identique à l'axe du corps de palier (4) à symétrie de rotation.

5. Dispositif selon l'une des revendications 2 à 4, **caractérisé en ce que** le corps de palier (4) à symétrie de rotation est d'une configuration cylindrique circulaire.

6. Dispositif selon l'une des revendications 2 à 4, **caractérisé en ce que** le corps de palier (4) à symétrie de rotation est d'une configuration sphérique.

7. Dispositif selon l'une des revendications 2 à 4, **caractérisé en ce que** le corps de palier (4) à symétrie de rotation est d'une configuration conique.

8. Dispositif selon l'une des revendications 2 à 7, **caractérisé en ce que** sur le corps de palier (4) à symétrie de rotation, est maintenu un support (5) qui porte les deux mors de serrage (10, 11).

9. Dispositif selon la revendication 8, **caractérisé en ce que** le support (5) est relié de manière rigide au corps de palier (4).

10. Dispositif selon la revendication 8, **caractérisé en ce que** le support (5) est maintenu sur le corps de palier (4) de manière à pouvoir tourner autour de l'axe de symétrie du corps de palier (4) à symétrie de rotation.

11. Dispositif selon la revendication 10, **caractérisé en ce que** le support (5) peut être emmanché, dans la direction de l'axe de rotation, sur le corps de palier (4) à symétrie de rotation.

12. Dispositif selon l'une des revendications précédentes, **caractérisé en ce que** l'un des deux mors de serrage est réalisé en tant que mors de serrage fixe (11), dont la distance à l'axe de rotation du corps d'excentrique (16) est sensiblement fixe, tandis que l'autre est réalisé en tant que mors de serrage mobile (10), qui peut être déplacé par le corps d'excentrique (16) vers le mors de serrage fixe (11).

13. Dispositif selon l'une des revendications 8 à 12, **caractérisé en ce que** le mors de serrage fixe (11) est relié de manière rigide au support (5).

14. Dispositif selon l'une des revendications 1 à 12, **caractérisé en ce que** sur le support (5) est disposée une butée (8) pour le mors de serrage fixe (11), qui limite un déplacement du mors de serrage fixe (11) dans la direction de serrage des mors de serrage (10, 11), et ainsi un éloignement du mors de serrage fixe (11) de l'axe de rotation du corps d'excentrique (16).

15. Dispositif selon l'une des revendications 8 à 12 ou 14, **caractérisé en ce que** les mors de serrage (10, 11) sont reliés au support (5) en pouvant pivoter autour d'un axe de rotation s'étendant parallèlement à la direction de serrage des mors de serrage (10, 11).

16. Dispositif selon la revendication 15, **caractérisé en ce que** l'axe de rotation des mors de serrage (10, 11) et l'axe de rotation du corps d'excentrique (16) se situent dans un même plan.

17. Dispositif selon la revendication 15, **caractérisé en ce que** l'axe de rotation des mors de serrage (10, 11) et l'axe de rotation du corps d'excentrique (16) sont décalés latéralement l'un par rapport à l'autre.

18. Dispositif selon l'une des revendications 15 à 17, **caractérisé en ce que** les deux mors de serrage (10, 11) sont formés par les parois latérales d'un corps de serrage (9) en forme de U en section transversale et dont les parois latérales peuvent fléchir de manière élastique l'une vers l'autre.

19. Dispositif selon l'une des revendications 15 à 18, **caractérisé en ce que** la liaison de pivotement entre les mors de serrage (10, 11) et le support (5) peut être interrompue.

20. Dispositif selon la revendication 19, **caractérisé en ce que** la liaison de pivotement comprend deux organes de palier s'engageant l'un derrière l'autre à la manière d'un système à baïonnette, et qui, dans une position angulaire déterminée, ne s'engagent plus l'un derrière l'autre et peuvent alors coulisser axialement l'un par rapport à l'autre.

21. Dispositif selon l'une des revendications précédentes, **caractérisé en ce qu'**entre le corps d'excentrique (16) et les mors de serrage (10, 11) est disposé un organe intermédiaire (18) qui, lors de la rotation du corps d'excentrique (16), est déplacé vers les mors de serrage (10, 11).

22. Dispositif selon la revendication 21, **caractérisé en ce que** l'organe intermédiaire (18) est maintenu mobile sur le support (5).

23. Dispositif selon la revendication 22, **caractérisé en ce que** l'organe intermédiaire (18) est maintenu sur le support (5) à l'aide d'une liaison élastique.

24. Dispositif selon l'une des revendications précédentes, **caractérisé en ce que** les mors de serrage (10, 11) peuvent, lorsque le corps d'excentrique (16) est débloqué, être écartés de manière élastique d'une façon suffisante pour qu'un élément de fixation (12) en forme de barre puisse s'enclencher entre eux.

25. Dispositif selon l'une des revendications 2 à 24, **caractérisé en ce que** le corps d'excentrique (16) est réalisé sous forme d'anneau.

26. Dispositif selon l'une des revendications précédentes, **caractérisé en ce que** le corps d'excentrique (16) présente des évidements (17) destinés à recevoir un outil de rotation.

27. Dispositif selon l'une des revendications 2 à 26, **caractérisé en ce que** le corps de palier (4) fait partie d'une vis (3) pour os.

28. Dispositif selon l'une des revendications 2 à 26, **caractérisé en ce que** le corps de palier (4) fait partie d'un crochet (36).

29. Dispositif selon l'une des revendications 2 à 26, **caractérisé en ce que** le corps de palier (4) est relié, par l'intermédiaire d'un support transversal (2), à une vis (3) pour os ou à un crochet (36), et **en ce que** l'axe longitudinal de la vis (3) pour os ou du crochet (36) s'étend de manière décalée latéralement par rapport à l'axe longitudinal du corps de palier (4).

30. Dispositif selon la revendication 29, **caractérisé en ce que** la vis (3) pour os ou le crochet (36) peuvent être tournés dans le support transversal (2) autour de leur axe longitudinal et peuvent être immobilisés par serrage dans une position angulaire déterminée.

31. Dispositif selon la revendication 30, **caractérisé en ce que** pour l'immobilisation par serrage, dans le support transversal (2) une pièce de serrage (27) peut coulisser contre la vis (3) pour os ou le crochet (36).

32. Dispositif selon la revendication 31, **caractérisé en ce que** pour le coulissement de la pièce de serrage (27), dans le support transversal (2) est monté un excentrique (26) appuyant sur la pièce de serrage (27) et pouvant tourner autour d'un axe de rotation.

33. Dispositif selon la revendication 32, **caractérisé en ce que** l'axe de rotation de l'excentrique (26) est perpendiculaire au plan du support transversal (2).

34. Dispositif selon la revendication 33, **caractérisé en ce que** l'axe de rotation de l'excentrique (26) s'étend parallèlement à l'axe longitudinal de la vis (3) pour os ou du crochet (36).

35. Dispositif selon l'une des revendications 32 à 34, **caractérisé en ce que** les axes de rotation de l'excentrique (26) dans le support transversal (2) et du corps d'excentrique (16) s'étendent parallèlement l'un à l'autre.

36. Dispositif selon la revendication 35, **caractérisé en ce que** les axes de rotation de l'excentrique (26) et du corps d'excentrique (16) sont confondus.

37. Dispositif selon la revendication 33, **caractérisé en ce que** la vis (3) pour os ou le crochet (36) est relié de manière pivotante au support transversal (2) par une liaison du type sphérique, et la pièce de serrage (27) peut être déplacée par coulissement, lors de la fixation, en direction d'une surface de palier sphérique (31) de la vis (3) pour os ou respectivement du crochet (36).

38. Dispositif selon l'une des revendications 31 à 37, **caractérisé en ce que** le support transversal (2) comporte un trou oblong (25) dans lequel sont logés la vis (3) pour os ou le crochet (36), la pièce de serrage (27) et l'excentrique (26).

39. Dispositif selon l'une des revendications 32 à 38, **caractérisé en ce que** l'axe de rotation de l'excentrique (26) et l'axe longitudinal du corps de palier (4) sont confondus.

40. Dispositif selon la revendication 39, **caractérisé en ce que** le corps de palier (4) est une douille reliée de manière fixe au support transversal (2), et à travers laquelle peut être inséré un outil (30) pour faire tourner l'excentrique (26).

41. Dispositif selon la revendication 40, **caractérisé en ce que** le corps de palier (4) présente des logements (33) pour un outil de retenue conjugué (34).

42. Dispositif selon la revendication 40 ou 41, **caractérisé en ce que** l'excentrique (26) est un anneau pouvant être tourné dans le support transversal (2) et disposé à l'extrémité, côté support transversal, du corps de palier (4).

43. Dispositif selon la revendication 40 ou 41, **caractérisé en ce que** l'excentrique (26) est disposé sur un noyau (32) monté rotatif dans le corps de palier (4), et s'étendant au moins sur une partie de sa longueur, à l'intérieur du corps de palier (4).

44. Dispositif selon la revendication 39, **caractérisé en ce que** le corps de palier (4) peut tourner autour de son axe longitudinal dans le support transversal (2), et porte lui-même l'excentrique (26).

45. Dispositif selon l'une des revendications 32 à 44, **caractérisé en ce que** l'excentrique (26) est fixé en direction axiale dans le support transversal (2).
